**Europäisches Patentamt**

(19) **European Patent Office**

**Office européen des brevets**

(11) Veröffentlichungsnummer: **0 044 489**
**B1**

(12) **EUROPÄISCHE PATENTSCHRIFT**

(45) Veröffentlichungstag der Patentschrift:
**14.12.83**

(51) Int. Cl.³: **A 61 B 5/10,** G 06 K 9/62

(21) Anmeldenummer: **81105403.0**

(22) Anmeldetag: **10.07.81**

(54) **Fingerabdrucksensor.**

(30) Priorität: **21.07.80 US 170606**

(43) Veröffentlichungstag der Anmeldung:
**27.01.82 Patentblatt 82/4**

(45) Bekanntmachung des Hinweises auf die Patenterteilung:
**14.12.83 Patentblatt 83/50**

(84) Benannte Vertragsstaaten:
**BE DE FR LU NL**

(56) Entgegenhaltungen:
**FR - A - 2 340 547**
**US - A - 3 781 855**
**US - A - 3 973 146**
**US - A - 4 250 894**

**IBM TECHNICAL DISCLOSURE BULLETIN, Band 23, Nr. 5, Oktober 1980, Seite 1837 New York, U.S.A. R.W. KOSS: "Mos chip probe sensor"**

(73) Patentinhaber: **SIEMENS AKTIENGESELLSCHAFT, Berlin und München Wittelsbacherplatz 2, D-8000 München 2 (DE)**

(72) Erfinder: **Rüll, Hartwig, Dr., Balduin-Helmstrasse 39, D-8080 Fürstenfeldbruck (DE)**

## Fingerabdrucksensor

Die vorliegende Erfindung bezieht sich auf einen Fingerabdrucksensor zum Umwandeln der Information, die in dem Fingerabdruck einer Person enthalten ist, in ein elektrisches Ausgangssignal.

Fingerabdruck-Identifikationssysteme, die den Abdruck eines Fingers, der auf eine Kontaktfläche gepresst wird, identifizieren können, sind an sich bekannt.

Aus US-PS Nr. 4053228 ist beispielsweise eine Fingerabdruck-Identifikationsapparatur bekannt, die eine transparente Glasplatte enthält, welche als eine Kontaktoberfläche oder ein Fingerabdruckleser dient. Ein Fingerabdruck wird durch Pressen eines Fingers gegen die Rückseite dieser Glasplatte und Halten des Fingers in einer vorbestimmten Position darauf gebildet. Der Fingerabdruck wird durch einen Lichtstrahl abgefragt, der durch die Vorderseite der Glasplatte eindringt. Der Abfragelichtstrahl wird teilweise an der Rückseite reflektiert, um einen Signallichtstrahl zu erzeugen, der die Information über den Fingerabdruck enthält. Der reflektierte Signallichtstrahl wird in Korrelation mit einem Hologramm desselben Fingerabdrucks gebracht, um die Identifikation durchzuführen.

Aus US-PS Nr. 4120585 ist ein anderes Fingerabdruck-Identifikationssystem bekannt. Dieses System enthält ein biegsames optisches Prisma als Fingerabdruckleser. Die Basis dieses Prisma wird körperlich durch den Finger der Person, die identifiziert werden soll, berührt. Das biegsame Prisma verformt sich unter dem zugeführten Druck. Es reflektiert teilweise einen Abtastlichtstrahl auf eine fotoempfindliche Einrichtung, die aktiviert wird. Die fotoempfindliche Einrichtung aktiviert ihrerseits weitere optische Komponenten des Fingerabdruck-Identifikationssystems. Auch in diesem Fall wird ein Fingerabdruckleser benutzt, der das Erhebungsvertiefungsmuster eines Fingerabdrucks der Person, die zu identifizieren ist, prüft.

In den beiden genannten US-Patentschriften wird der Fingerabdruck entweder auf einer Glasplatte oder auf der Basis eines Glasprisma ausgebildet. Der Fingerabdruck wird augenblicklich durch Einstrahlung von Licht überprüft. Es ist weder eine mechanische noch eine elektrische Speicherung des Fingerabdrucks vorgesehen. Allerdings ist es ratsam, die Information des Fingerabdruckmusters in einen Rechner einzulesen. Der Rechner kann dann eine Überprüfung unter Berücksichtigung besonderer Merkmale und unter Berücksichtigung der Identifikation im Zusammenhang mit einigen gespeicherten Informationen vornehmen. Deshalb besteht ein Bedarf, die Fingerabdruckinformation einer Person in einen Rechner einzugeben. Das erfordert einen Fingerabdrucksensor zum Umwandeln der Fingerabdruckinformation in ein elektrisches Ausgangssignal, das in einen Rechner eingegeben werden kann.

In dem Artikel „Piezoelectric and Pyroelectric Polymer Sensors", Proc. Conf. on Sensor Tech-nology for Battlefield and Physical Security Applications, Fort Belvoir, VA, 13.-15. Juli, 1977, S. 204-212 (US Army Mobility Equipment Research and Development Command, Fort Belvoir, VA, Juli 1977) sind synthetische organische Polymere offenbart, die piezoelektrische Eigenschaften besitzen. Piezoelektrische Polymere sind in Form von dünnen Schichten auf dem Markt erhältlich. Sie sind nachgebend, flexibel, zäh, leicht und relativ billig. Sie können ausserdem leicht auf einer Oberfläche aufgebracht werden. Dazu können Gumilösung, Zyanakrylat, Epoxidharze oder andere Kleber benutzt werden. Eines der im allgemeinen benutzten piezoelektrischen Polymere ist Polyvinylidenfluorid (PVDF). Der piezoelektrische Modul dieses Polymers ist etwa sechsmal so gross wie der eines typischen piezoelektrischen Keramikwerkstoffes, wie beispielsweise Bleizirkonat oder Titanatkeramik. Ein piezoelektrisches Polymer stellt seine starken piezoelektrischen Eigenschaften durch Erhitzen des Materials, Anlegen eines starken elektrischen Feldes und Herunterfahren auf Raumtemperatur mit dem angelegten elektrischen Feld zur Verfügung. Ein solcher Vorgang resultiert in der Ausrichtung einer erheblichen Anzahl von elektrischen Dipolen senkrecht zu der Ebene des Plättchens. Jede Anregung, die die Dikke des Plättchens verändert, verändert ebenso die Oberflächenladungsdichte auf jeder der Flächen. Eine mehr ins Detail gehende Analyse ist in „J. Appl. Phys." 46, 4204 (1975) veröffentlicht. Es können entweder die Ladung, ein Kurzschlussstrom oder eine Leerlaufspannung gemessen werden, um beispielsweise den Druck auf eine Plättchen aus piezoelektrischem Polymer zu bestimmen. Die Flexibilität, das geringe Gewicht und die Freiheit von Ermüdung von Polymeren lässt sie zum Messen verschiedener physikalischer Parameter, einschliesslich Drücken geeignet sein. Die Dünnheit und Flexibilität von Polymermesssonden lässt sie sehr ähnlich der Haut sich anfühlen und mechanisch wirken. Daher können polymere Messsonden als Bandagierungsmittel angewendet werden, um beispielsweise Herztöne und Pulsraten von Patienten während deren Untersuchung zu überwachen. Bestimmte Anordnungen von Messsonden können für eine akustische Holografie benutzt werden. Der aktive Bereich einer Messsonde kann auf jede geeignete Grösse und Form, wie sie für die Messung benötigt werden, zugeschnitten werden. In Übereinstimmung mit der Druckschrift „Piezoelectric and Pyroelectric Polymers", Supra, und in Übereinstimmung mit US-PS Nr. 3970862 besteht ein typischer Sensor zum Messen von Temperatur oder Druck aus einem mehrschichtigen Aufbau (sandwich) von zwei dünnen Polymerplättchen. Jedes Plättchen weist aufgedampfte Metallelektroden auf beiden Seiten auf. Die Plättchen werden derart miteinander verklebt, dass Ladungen derselben Polarität auf den inneren Flächen auftreten. Der Mittelleiter eines Koaxialkabels wird mit den Elektroden dieser inneren Flächen und der Schirm des Kabels mit den Elektroden der äusseren Flächen verbunden. Auf diese Weise liegen alle nach aussen gerichteten Oberflächen auf Erdpotential, und das Signal aus dem Inneren des Sensors ist gut abgeschirmt. Allerdings sind Sensoren zum Messen der Verteilung von Drücken nicht in dem Artikel in „J. Appl. Phys." offenbart.

In der technischen Hilfsbroschüre „Transducer With A Sense of Touch" des JET Propulsion Laboratory, California Institute of Technology, Pasadena, California, November 1979, ist ein Berührungs- oder Drucksensor offenbart, der die Form und die Druckverteilung eines Objekts, das in Berührung mit seiner Oberfläche steht, zu bestimmen in der Lage ist. Die Sensorausgangssignale können als ein Feld von alphanumerischen Symbolen auf dem Bildschirm eines Videomonitors angezeigt oder dazu benutzt werden, eine Druck-„Landkarte" der Oberfläche des Objekts zu entwickeln. Die Signale können ausserdem mechanische oder elektrische Einrichtungen steuern. Der Berührungssensor besteht aus einer Matrix kleiner Elektroden in einem Metallrahmen, welcher mit einer Schicht eines druckleitenden Plastikmaterials überlegt ist. Der Rahmen, der aus vielen Zellen besteht, wird auf Erdpotential gehalten, wogegen eine gemeinsame Stromquelle an die Elektroden angeschlossen ist, die in den Zellen vorgesehen sind. Ein Druck auf die Plastikschicht verändert die Leitfähigkeit des Pfades zwischen einer Elektrode und dem metallischen Rahmen. Aus diesem Grunde erzeugt der Strom, der durch die Elektrode fliesst, eine Messspannung des Druckes über einen Widerstand in Reihe mit der Elektrode. Die Spannung in der Matrix liefert Information über die Form und Oberflächenkonturen des Objekts, das die Plastikschicht berührt. Der offenbarte Wandler ist kein Fingerabdrucksensor im Sinne der vorliegenden Erfindung. Fingerabdrücke sollen weder erkannt noch bewertet werden. Die Aufgabe besteht vielmehr darin, eine „mechanische Hand", etwa für einen Industrieroboter, mit einem Berührungssensor auf einem oder mehreren ihrer Finger ausstatten zu können.

Es sind auch Fingerabdrucksensoren bekannt, bei denen die Information des Fingerabdruckes direkt aus einer mechanischen Information (Druckeinwirkung auf die Kontakteinrichtung) in eine elektrische Information (Ausgangssignal der elektrischen Einrichtung) umgewandelt wird. In der FR-A Nr. 2340547 ist ein derartiger Fingerabdrucksensor beschrieben, der die Erhebungen des Fingers nachweist durch Messung eines Stromes, der beim Anpressen des Fingers an eine Kontaktfläche einer Kontakteinrichtung zwischen den Erhebungen des Fingers und in der Kontaktfläche angeordneten Sensoren fliesst. In US-A Nr. 3781855 ist ein ähnlicher Fingerabdrucksensor beschrieben, bei dem die Leitfähigkeit zwischen Kontaktpaaren gemessen wird, die in der Oberfläche der Kontakteinrichtung angeordnet ist, wobei der Hautkontakt zwischen den Kontaktpaaren für die unterschiedliche Leitfähigkeit verantwortlich ist.

Der vorliegenden Erfindung liegt die Aufgabe zugrunde, einen verbesserten Fingerabdrucksensor zum direkten Umformen der Fingerabdruckinformation eines berührenden Fingers in ein elektrisches Ausgangssignal zu schaffen. Ein solcher Fingerabdrucksensor soll das elektrische Ausgangssignal, das die Informationen repräsentiert, die in einem Fingerabdruck enthalten sind, in einen Rechner für die weitere Verarbeitung einlesen können. Der Fingerabdrucksensor soll einen einfachen Aufbau haben, billig herzustellen sein und eine hohe Empfindlichkeit und eine hohe Zuverlässigkeit aufweisen. Des weiteren soll der zu schaffende Fingerabdrucksensor aus auf dem Markt leicht erhältlichen Komponenten zusammengesetzt werden können. Der Fingerabdrucksensor soll auch unempfindlich gegenüber Störsignalen und insbesondere unabhängig gegenüber Temperatureinflüssen sein.

Weitere Aufgabenstellungen werden aus der im folgenden gegebenen Beschreibung ersichtlich.

Die der Erfindung zugrundeliegende Aufgabe wird durch einen Fingerabdrucksensor gemäss dem Oberbegriff des Patentanspruchs 1 gelöst, der durch die im kennzeichnenden Teil des Patentanspruchs 1 angegebenen Merkmale charakterisiert ist.

Die Kontakteinrichtung ist vorzugsweise aus einem flexiblen piezoelektrischen Polymer hergestellt. Sie kann allerdings auch aus einem piezoelektrischen Keramikwerkstoff hergestellt sein. Falls ein Polymer benutzt wird, sollte vorzugsweise das wohlbekannte piezoelektrische Polymer Polyvinylidenfluorid (PVDF) oder ein verwandtes Material zur Anwendung gelangen. Falls ein Keramikwerkstoff benutzt wird, kann ein Bleizirkonattitanatkeramikwerkstoff angewendet werden, beispielsweise einer, der Bariumtitanat oder Triglyzinsulfat enthält.

Das piezoelektrische Material kann derart strukturiert sein, dass es eine Anordnung einer Matrix von erhabenen Segmenten enthält, um ein „Übersprechen" zwischen benachbarten Vertiefungen des Fingerabdrucks zu vermeiden. Die Auslegung in Segmenten kann isotropisch oder nichtisotropisch sein.

Die elektrische Einrichtung zur Bestimmung der Ladungen kann vorzugsweise eine Ladungsleseeinrichtung sein, wie sie in einer ladungsgekoppelten Einrichtung (CCD) benutzt wird. Beispielsweise werden üblicherweise CCD-Technologien angewendet, um die Ladungsverteilung in CCD-Fotodetektoranordnungen abzutasten. In diesem Zusammenhang sollte erwähnt werden, dass kürzlich eine CCD-Matrix mit $2 \times 10^5$-Segmenten auf einer Fläche von wenigen Quadratzentimetern hergestellt worden ist. Dies zeigt deutlich, dass die CCD-Technologie weit genug entwickelt worden ist, um ein Scanning mit einer hohen Auflösung einer räumlich modulierten Ladungsverteilung, wie sie durch einen Fingerabdruck erzeugt wird, durchzuführen. Einzelheiten von CCD-Einrichtungen sind beispielsweise in einem Datenbuch der Bezeichnung „Solid-State Image Sensor Array

RA 100×100" von EG & G. Reticon, Sunnyvale, California 94086, beschrieben.

Im Betrieb des Fingerabdrucksensors wird der zu untersuchende Finger gegen die Kontaktoberfläche gepresst. Die Erhebungen des Fingerreliefs üben einen bestimmten Betrag eines Druckes auf die piezoelektrische Substanz aus. In den Regionen der Erhebungen werden lokale Ladungen aufgebaut, während in den Regionen der Vertiefungen keine Ladungen oder kleinere lokale Ladungen erzeugt werden. Dies bedeutet, dass die Geometrie der Hautstruktur des Fingers in eine räumlich variiende Ladungsverteilung umgesetzt wird. Diese Ladungsverteilung wird erkannt und gemessen, vorzugsweise durch Einrichtungen mit den erwähnten Technologien für ladungsgekoppelte Einrichtungen (CCD).

Die einfache Konstruktion des neuen Fingerabdrucksensors bedient sich aller Vorteile des piezoelektrischen Materials. Für den Fall, dass ein piezoelektrisches Polymer als Kontakteinrichtung oder als Abtastelement verwendet wird, kann eine flexible Schicht oder ein flexibles Plättchen angewendet werden. Eine solche Schicht ist leicht und billig und stellt einen grossen dynamischen Bereich und eine aussergewöhnliche Empfindlichkeit zur Verfügung. Ein solches Polymer ist schwerlich durch Umweltbedingungen wie Salzwasser, Seife, allgemeine organische Lösungen, mechanische Schocks usw. zu beschädigen.

Da beim vorliegenden Fingerabdrucksensor, wie zuvor erwähnt, die CCD-Technologie benutzt werden kann, ist es möglich, den Fingerabdrucksensor als Festkörperwandler aufzubauen. Demzufolge kann der Sensor wie eine Festkörperkomponente gefertigt werden und wird auch die Zuverlässigkeit einer solchen haben. Die Sensorelemente können sehr klein sein. Sie sind billig und zuverlässig und benötigen keinerlei Wartung.

Ein Fingerabdrucksensor gemäss der vorliegenden Erfindung ist sehr gut als steckbarer Modul für verschiedene Systeme, wie beispielsweise ein Türschloss oder ein Rechnerendgerät, zu verwenden.

Im folgenden wird die Erfindung anhand mehrerer Ausführungsbeispiele für die Erfindung betreffender Figuren im einzelnen erläutert.

Fig. 1 zeigt eine perspektivische Ansicht eines Ausführungsbeispiels für einen Fingerabdrucksensor gemäss der vorliegenden Erfindung mit einer Kontakteinrichtung, die ein piezoelektrisches polymeres oder keramisches Material enthält, und mit einer Sensormatrix zur Messung oder Bestimmung von elektrischen Ladungen auf dem piezoelektrischen Material.

Fig. 2 zeigt schematisch ein Blockschaltbild mit einem Querschnitt durch ein weiteres Ausführungsbeispiel für den erfindungsgemässen Fingerabdrucksensor, wobei eine Kontakteinrichtung und eine Ladungsmesseinrichtung mit zahlreichen Elektroden vorgesehen sind.

Fig. 3 zeigt einen Querschnitt durch eine Kontakteinrichtung gemäss Fig. 2, auf die Druck durch einen Finger ausgeübt wird.

Fig. 4 zeigt einen Teilquerschnitt eines weiteren Ausführungsbeispiels für die Kontakteinrichtung.

Fig. 5 zeigt einen Teilquerschnitt durch ein anderes Ausführungsbeispiel für die Kontakteinrichtung.

Fig. 6 zeigt eine perspektivische Ansicht eines weiteren Ausführungsbeispiels für den erfindungsgemässen Fingerabdrucksensor mit einer Kontakteinrichtung, die aus piezoelektrischem polymeren oder keramischen Material aufgebaut ist, das in Form einer Matrix strukturiert ist und eine Sensormatrix zum Bestimmen der Verteilung der Ladungen darstellt.

Fig. 7 zeigt einen Querschnitt durch ein weiteres Ausführungsbeispiel einer strukturierten Kontakteinrichtung.

Fig. 8 zeigt schematisch einen Fingerabdruck.

Fig. 9 zeigt die Draufsicht einer Sensoroberfläche, die erhabene Segmente hat, welche in Übereinstimmung mit der Struktur eines Fingerabdrucks angeordnet sind.

Fig. 10 zeigt schematisch eine Ansicht einer Anordnung von unterschiedlichen Elektroden.

Fig. 11 zeigt einen flachen Fingerabdrucksensor, der Emitter und Empfänger hat, die über dessen Rand verteilt angeordnet sind.

Fig. 12 zeigt eine perspektivische Ansicht eines eingewölbten Fingerabdrucksensors.

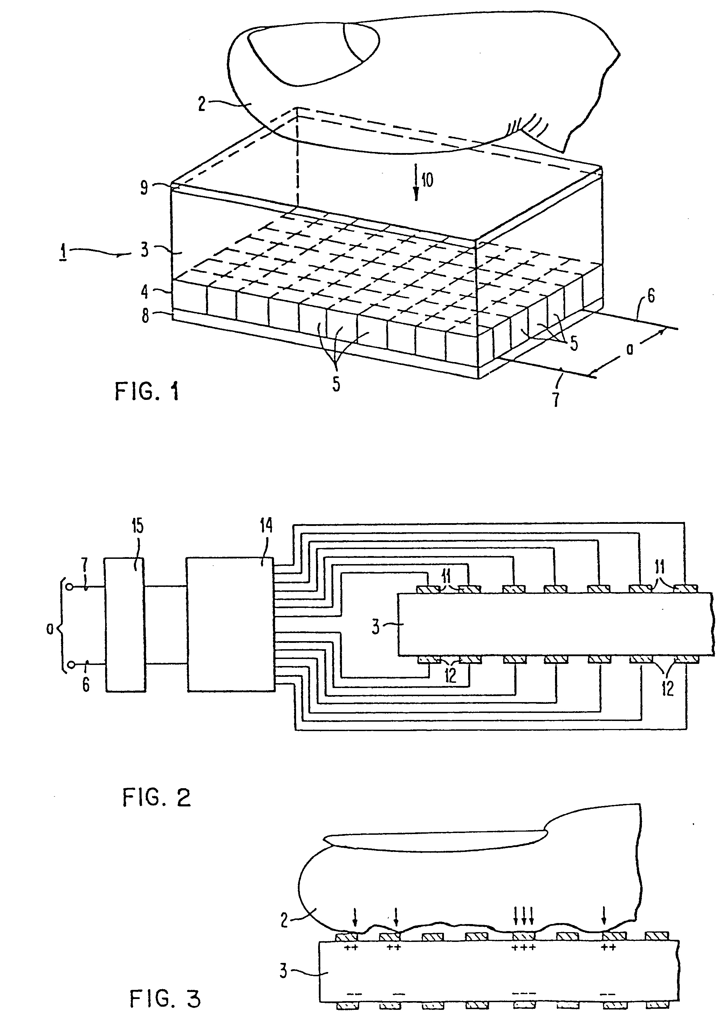

Wie bereits erläutert, zeigt Fig. 1 einen Fingerabdrucksensor 1 zum Umsetzen der in dem Fingerabdruck eines Fingers 2 enthaltenen Information in ein elektrisches Ausgangssignal a. Der Fingerabdrucksensor 1 tastet die Verteilung der Erhebungen und Vertiefungen der Haut der Fingerkuppe ab. Er enthält zwei Hauptkomponenten, nämlich eine Kontakteinrichtung 3 und eine elektrische Ladungsmesseinrichtung 4.

Die Kontakteinrichtung 3 besteht aus einem homogenen piezoelektrischen Material, beispielsweise aus piezoelektrischem Keramikmaterial oder vorzugsweise einem flexiblen piezoelektrischen Polymer, beispielsweise Polyvinylidenfluorid (PVDF). Sie hat die Form einer rechteckigen Platte oder Schicht. Auf der Unterseite der Kontakteinrichtung 3 ist die Ladungsmesseinrichtung 4 angeordnet. Die Ladungsmesseinrichtung 4 besteht aus einer Anordnung einer grossen Anzahl von Sensorelementen 5. Diese Sensorelemente 5 erlauben das Messen der elektrischen Ladung in Bereichen, die so klein oder sogar kleiner als die Erhebungen und Vertiefungen in der Fingerkuppenhaut des Fingers 2 sind. Dazu sind in Wirklichkeit mehr Sensorelemente 5 als gezeigt vorgesehen. In dem Ausführungsbeispiel gemäss Fig. 1 sind die Sensorelemente 5 in Form einer rechteckigen Sensormatrix angeordnet. Wie weiter unten erklärt wird, können ebenfalls andere Anordnungen gewählt werden. Die Sensormatrix kann an der Kontakteinrichtung mittels eines Klebers angebracht sein. Sie kann ebenfalls auf der unteren Fläche als ein Film aufgedampft sein oder mit Hilfe eines galvanischen Prozesses aufgebracht werden. Abhängig von der Art der Ladungsmesseinrichtung, die verwendet wird, können ebenfalls weitere Zusatzeinrichtungen vorgesehen sein.

Fig. 1 kann entnommen werden, dass zwei Ausgangsleitungen, nämlich eine erste Ausgangsleitung 6 und eine zweite Ausgangsleitung 7 mit der Ladungsmesseinrichtung 4 verbunden sind, um das elektrische Ausgangssignal a, welches mit dem Relief des Fingerabdrucks korrespondiert, abnehmen zu können.

In einem speziellen Ausführungsbeispiel kann die Unterseite der Ladungsmesseinrichtung 4 an einer Grundplatte 8 aus einem isolierenden Material angebracht sein.

Die obere Seite der Kontakteinrichtung 3 kann mit einer sehr dünnen Abdeckschicht 9 versehen sein, die die Aufgabe einer Schutzumhüllung oder einer Elektrode zum Bestimmen anderer Parameter darstellen kann. Eine derartige Elektrode kann beispielsweise dazu benutzt werden, um die Temperatur der Kontakteinrichtung zu prüfen. Ebenso kann eine solche Elektrode dazu benutzt werden, Informationen über den Pulsschlag der Person, die die Oberfläche der Einrichtung berührt, zu gewinnen. In anderen Worten: Die Information kann zusätzlich zur Erstellung eines EKG für weitere Untersuchungen verwendet werden.

Sobald der Finger 2 die Oberseite der Kontakteinrichtung 3 berührt und einen Druck auf diese in Richtung eines Pfeiles 10 ausübt, wird eine inhomogene Verteilung von elektrischen Bipolen in der Kontakteinrichtung 3 hervorgerufen. Die Achsen dieser Bipole sind senkrecht zu der ebenen Oberfläche ausgerichtet. Die Verteilung der elektrischen Ladungen über die Fläche stellt eine exakte Kopie der Struktur des Fingerabdrucks dar. Das bedeutet, dass Stellen mit einer niedrigen Dichte von Ladungen mit den Vertiefungen der Oberfläche der Struktur der Kuppe des Fingers 2 korrespondieren, wogegen Stellen mit hohen Elektronenladungen mit Erhebungen in der Oberflächenstruktur des Fingers 2 korrespondieren. Die Verteilung der Stellen mit hohen und niedrigeren Ladungen wird durch die Ladungsmesseinrichtung 4 abgetastet. Jedes Sensorelement 5 gibt ein Signal korrespondierend mit der Ladung des Segments des piezoelektrischen Materials, dem diese zugeordnet ist, ab. Je mehr Sensorelemente 5 vorgesehen sind, desto besser und genauer ist die Information über den Fingerabdruck. Das bedeutet: Je mehr Sensorelemente benutzt werden, desto höher ist die Auflösung.

Für das Ausführungsbeispiel gemäss Fig. 1 ist angenommen, dass die Ladungsmesseinrichtung 4 vorzugsweise eine Ladungsausleseeinrichtung in CCD-Technologie ist. Es können jedoch auch andere Ladungsmesseinrichtungen 4 verwendet werden, beispielsweise eine Ladungsmesseinrichtung 4, die die Ladungsverteilung durch Messen der Ladungen der Sensorelemente 5 in einem Scan-Vorgang Zeile für Zeile abtastet.

Fig. 2 zeigt, wie bereits erläutert, ein weiteres Ausführungsbeispiel für den erfindungsgemässen Fingerabdrucksensor. In diesem Ausführungsbeispiel wird eine andere elektrische Schaltkreistechnik zum Messen der Verteilung von Ladungen auf den Oberflächen der Kontakteinrichtung 3 benutzt. Die Kontakteinrichtung 3, die wiederum aus einem piezoelektrischen Material hergestellt ist, ist auf ihrer Oberseite mit einer Struktur oder einer Anordnung von kleinen metallischen oberen Elektroden 11 und auf ihrer Unterseite mit einer ähnlichen Struktur oder Anordnung von kleinen metallischen unteren Elektroden 12 versehen. Paare von Elektroden 11 bzw. 12 sind sich gegenüberliegend angeordnet. Die Elektroden 11 und 12 sind über Verbindungsleitungen an einen Miltiplexer 14 angeschlossen, der seinerseits mit einer Ladungsauswerteeinrichtung 15 verbunden ist. Das elektrische Ausgangssignal a wird über die Ausgangsleitungen 6, 7 der Ladungsauswerteeinrichtung 15 abgegeben. In diesem Ausführungsbeispiel ist jedes der Sensorelemente 5 (vgl. Fig. 1) durch ein spezielles Paar von Elektroden 11, 12 realisiert, das zum Messen durch den Multiplexer 14 und die Ladungsauswerteeinrichtung 15 abgetastet wird. Nachdem nur eine einzige Ladungsauswerteeinrichtung 15 vorgesehen ist, werden die Elektroden 11, 12 seriell durch den Multiplexer 14 abgefragt oder gescannt. Es ist auch möglich, nur eine einzige Gruppe von allen Elektroden 11 und 12 durch einen Multiplexer und eine andere Gruppe durch einen anderen Multiplexer abzuscannen.

In Fig. 3 ist gezeigt, dass der Finger 2 mit einem räumlich variierenden Druckfeld in Übereinstimmung mit den Erhebungen und Vertiefungen der Hautoberfläche auf die Kontakteinrichtung 3 einwirkt. Dieses Druckfeld verursacht eine örtlich sich ändernde Ladungsverteilung, wie es in Fig. 3 durch einige Plus- und Minussymbole dargestellt ist. Die Ladungen können nicht abfliessen, da die Kontakteinrichtung 3 einen Isolator darstellt.

Fig. 4 zeigt ein weiteres Ausführungsbeispiel für den Fingerabdrucksensor. Es ist ersichtlich, dass der Querschnitt durch die oberen Elektroden 11 jeweils ein auf den Kopf gestelltes T darstellt, während der Querschnitt durch die unteren Elektroden 12 jeweils rechteckförmig ist. Diese zuletzt genannten Elektroden 12 sind flache Scheiben. Wie ferner gezeigt ist, ist in diesem Ausführungsbeispiel ein elektrisch schwach leitendes dielektrisches Füllmaterial 17 bzw. 18 zwischen jeweils sich gegenüberstehenden Elektroden 11 und 12 vorgesehen. Das Einbringen dieses dielektrischen Füllmaterials 17, 18 verhindert Leckströme. Zusätzlich zu diesem Effekt sorgt das dielektrische Füllmaterial 17, 18 für eine gute mechanische Festigkeit der Elektrodenanordnungen.

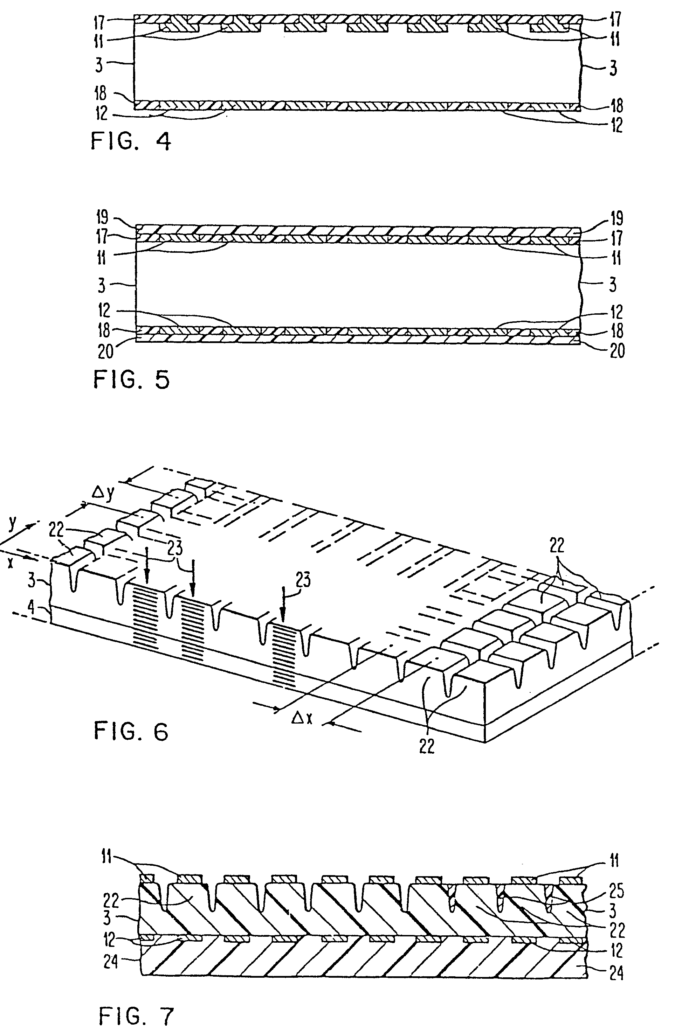

Fig. 5 zeigt, wie bereits erläutert, ein weiteres Ausführungsbeispiel für einen Fingerabdrucksensor gemäss der vorliegenden Erfindung. In diesem Ausführungsbeispiel sind ebenfalls Elektroden 11 und 12 für den Sensor auf beiden Seiten der Kontakteinrichtung vorgesehen. Die Elektroden 11, 12 sind entweder als kleine zylindrische Scheiben oder als kleine rechteckförmige Elemente ausgeführt. Auch in diesem Ausführungsbeispiel ist ein dielektrisches Füllmaterial 17, 18 zwischen die Elektroden 11 bzw. 12 eingebracht. Dünne Folien 19, 20 schützen die Elektroden 11 bzw. 12 und das dielektrische Füllmaterial 17 bzw. 18 vor Schmutz und Abnutzung. Die Folien 19 und 20 bestehen jeweils aus einem dielektrischen Material. Die obere Folie 19 ist vorzugsweise eine druckleitende Plastikfolie, die in der Lage ist, den Druck des Fingers ohne jede Abschwächung in Richtung des Pfeiles, der auf die Elektrode 11 weist, zu übertragen. Die obere Schutzfolie, nämlich die Folie 19, kann aus Mylar (eingetr. WZ) bestehen. Die untere Folie 20, die nur eine Schutzfolie darstellt, kann aus einem billigeren Plastikmaterial bestehen.

Fig. 6 und 7 zeigen weitere Versionen eines Fingerabdrucksensors gemäss der vorliegenden Erfindung. Diese Fingerabdrucksensoren enthalten jeweils ebenfalls eine Kontakteinrichtung 3 und eine Ladungsmesseinrichtung 4, welche in Fig. 6 die Form einer Sensormatrix hat. In diesen beiden Versionen enthält die obere Seite der Kontakteinrichtung 3 eine bestimmte Struktur. Diese Oberseite ist ähnlich wie eine Schokoladentafel strukturiert. Das heisst, dass die Oberfläche eine Vielzahl von erhabenen Segmenten 22 enthält. Diese Segmente 22 haben entweder eine rechteckförmige oder eine runde Form. Sie sind voneinander durch „Gräben" getrennt, deren Tiefe etwa gleich der Hälfte der Dicke der Kontakteinrichtung 3 sein kann. Die erhabenen Segmente 22 können eine rechteckförmige Matrix bilden. Die Grösse dieser Matrix entspricht in Fig. 6 der Sensormatrix der elektrischen Ladungsmesseinrichtung 4. Das Vorsehen der Segmente 22 verhindert ein „Übersprechen" zwischen benachbarten Bereichen, das heisst, dass die „Gräben" einen gewissen Grad von Ladungstransport von einem Segment 22 zu den benachbarten Segmenten 22 verhindern.

In dem Querschnitt gemäss Fig. 7 ist gezeigt, dass eine Ladungsmesseinrichtung 4 vorgesehen ist, die Elektroden 11 und 12 aufweist. Die unteren Elektroden 12 sind in eine Isolationsträgerplatte 24 eingebettet, die an der Kontakteinrichtung 3 angebracht ist.

Wie auf der rechten Seite von Fig. 7 gezeigt ist, können die „Gräben" mit einem geeigneten nichtleitenden Material 25 aufgefüllt sein. Durch die Verwendung eines derartigen nichtleitenden Materials 25 kann die Isolation zwischen benachbarten Segmenten 22 verbessert werden.

In Fig. 6 ist gezeigt, dass in Übereinstimmung mit den Vertiefungen und Erhebungen in der Struktur der Fingerkuppe ein mehr oder weniger wirksamer Druck auf die Segmente 22 ausgeübt wird. Es sei angenommen, dass drei seitliche Segmente 22 einem wirksamen Kontaktdruck durch einige Erhebungen in der Fingerkuppenhaut ausgesetzt sind. Die Druckkräfte sind durch drei Pfeile 23 angedeutet. Ein Druck in Richtung der Pfeile 23 resultiert in einer elektrischen Ladung auf den gegenüberliegenden Flächen der drei Segmente 22, welche hier betrachtet werden. In dem Ausführungsbeispiel gemäss Fig. 6 werden die Ladungen dieser drei Matrixelemente der Ladungsmesseinrichtung 4, die zu den Segmenten 22 gehören, welche unter Druck stehen, erkannt. Aus der perspektivischen Darstellung in Fig. 6 ist ersichtlich, dass sich der Fingerabdrucksensor in zwei Richtungen x bzw. y erstreckt, die senkrecht zueinander verlaufen. Von Wichtigkeit bei der Auslegung des Fingerabdrucksensors ist die Wahl der Proportionen der Segmente 22 und deren gegenseitigen Abstände. Einerseits sollten die Segmente 22 eine Fläche haben, die so gross wie möglich ist, um eine hohe Druckempfindlichkeit zu erzielen. Andererseits sollten die Segmente 22 eine bestimmte Grösse nicht überschreiten, da die örtliche Auflösung des Fingerabdrucksensors dann zu schlecht würde und folglich feine Strukturen in einem Fingerabdruck nicht erkannt werden könnten. Solche feinen Strukturen treten beispielsweise oft in den Fingerabdrücken orientalischer Frauen auf.

Für den Abstand $\Delta x$, $\Delta y$ zwischen benachbarten Segmenten 22 gilt die folgende Bedingung:

$$\Delta x, \Delta y \leq d \qquad (1)$$

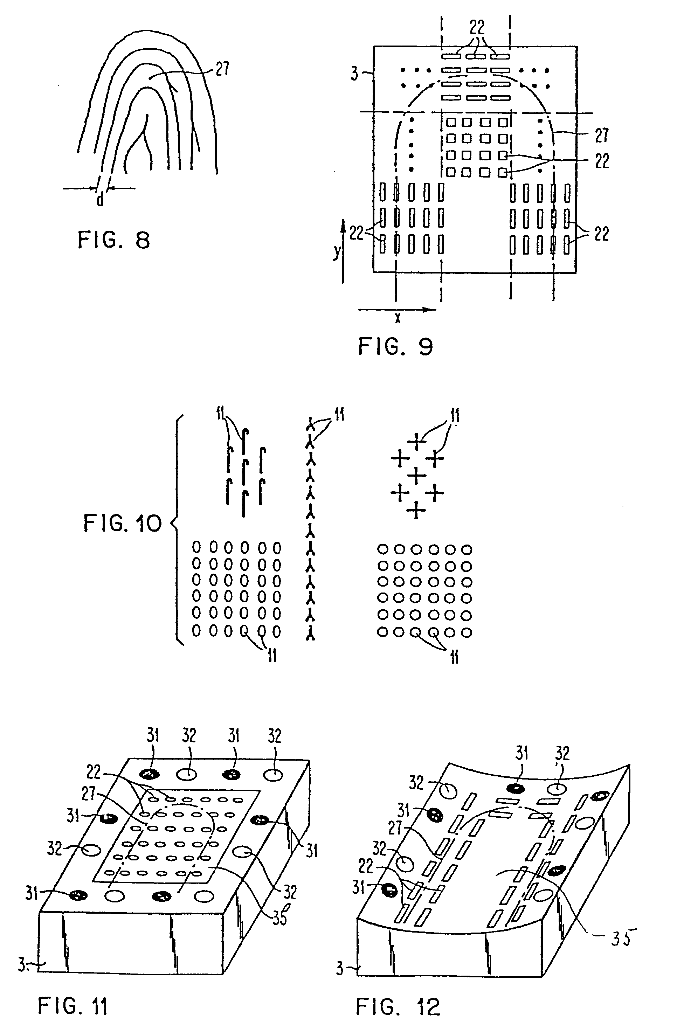

Diese Bedingung sollte wegen des Abtasttheorems beachtet werden. Die Grösse d kennzeichnet in dieser Bedingung das kleinste Detail, das durch den Fingerabdrucksensor erkannt werden soll. Entsprechend dem Fingerabdruck gemäss Fig. 8 ist die Grösse d die kleinste Entfernung zwischen den Vertiefungen des Fingerabdrucks.

Im Falle einer Struktur von in gleichen Abständen voneinander angeordneten erhabenen Segmenten 22 gilt die folgende Bedingung:

$$\Delta x = \Delta y = d/2 \qquad (2)$$

Diese Bedingung sollte beachtet werden und zwar in bezug auf das räumliche Abtasttheorem.

Fig. 9 zeigt, wie bereits erwähnt, die Draufsicht eines Fingerabdrucksensors, der eine Vielzahl von erhabenen Segmenten 22 aufweist. Es sind jedoch nur wenige Gruppen aus der Vielzahl der erhabenen Segmente 22 gezeigt, damit die Figur übersichtlich bleibt. Wie aus Fig. 9 zu erkennen ist, müssen die erhabenen Segmente keinesfalls notwendigerweise eine quadratische Form haben, und die Anordnung muss nicht notwendigerweise derart getroffen sein, dass alle erhabenen Segmente 22 jeweils die gleiche Entfernung voneinander aufweisen. Wie gezeigt, können Form und Anordnung der erhabenen Segmente 22 zumindest bis zu einem gewissen Grad und in bestimmten Gruppen an die Struktur der Erhebungen und Vertiefungen in der Haut des menschlichen Fingerabdrucks angepasst sein.

An der linken und rechten Seite eines abgenommenen Fingerabdrucks 27 sind die Vertiefungen und Erhebungen ungefähr parallel zu einer senkrechten y-Achse orientiert. An dem oberen Ende des Strukturfeldes des Fingerabdrucks verlaufen die Vertiefungen und Erhebungen horizontal, d.h. parallel zu einer x-Achse. Die erhabenen Segmente 22 (zusammen mit den Elektroden 11 und 12) an der linken und an der rechten Seite und an dem oberen Ende sind rechteckförmig ausgebildet. Sie folgen mit ihren Längsachsen der Struktur der Erhebungen und Vertiefungen.

Im Zentrum oder Kern des Fingerabdrucks 27 wird eine hohe Auflösung gefordert. In diesem zentralen Bereich sind kleinere erhabene Segmente 22 und kleinere Elektroden 11 und 12 mit einer hohen Packungsdichte vorgesehen. Diese erhabe-

nen Segmente können eine quadratische Oberfläche besitzen.

In Fig. 10 sind verschiedene Ausführungsformen der Elektroden 11 und/oder der erhabenen
Segmente 22 gezeigt. Damit übereinstimmend
können die Elektroden 11 kreisförmige oder elliptische Scheiben sein, sie können die Form eines L,
eines Sterns, eines Kreuzes usw. haben.

Von Bedeutung ist, dass die Elektroden 11 nicht
notwendigerweise in Form eines Schachbrettmusters angeordnet sein müssen. Im Gegenteil
können andere geeignete Strukturen ausgewählt
werden, die an die Oberflächenstruktur des menschlichen Fingerabdruckes angepasst sind. Es sind
auch konzentrische Ringe, Spiralen und andere
Formen anwendbar. Die Elektroden 11 können
längs des Berührungsfeldes eines „Standard-Fingerabdrucks" angeordnet sein.

In Fig. 11 ist eine rechteckförmige Anordnung
von Segmenten 22 für den Sensor gezeigt, die von
einem Rahmen umgeben ist, welcher verschiedene Paare von Infrarot-(IR)-Sendern 31 und In-
frarot-(IR)-Empfängern 32 enthält. Die Infrarot-
(IR)-Sender 31 geben vorzugsweise Strahlung
nahe dem Infrarotbereich ab. Die Infrarot-(IR)-
Sender 31 können lichtemittierende Dioden LED
und die Infrarot-(IR)-Empfänger 32 können Fotodioden sein. Beide Komponenten haben nur kleine
Abmessungen und können leicht in dem Rahmen
in der Nähe der Sensoroberfläche untergebracht
werden. Sobald ein Finger die Sensoroberfläche 35 berührt, wird die Infrarotstrahlung, die entweder impulsförmig oder konstant abgegeben
wird, in das Innere des Fingers geleitet. Die durch
den Finger empfangene Strahlung wird durch einen koordiniert arbeitenden Infrarot-(IR)-Empfänger 32 empfangen und in ein elektrisches Signal, beispielsweise elektrische Impulse, umgesetzt.

Die Infrarot-(IR)-Sender 31 und die Infrarot-
(IR)-Empfänger 32 können zwei Funktionen haben: Sie können Teil einer Positionserkennungs-
und Signalisierungseinrichtung und gleichzeitig
Teil eines Detektors für Artefakte sein.

Die Infrarot-(IR)-Sender 31 und die Infrarot-
(IR)-Empfänger 32 sind mit einer ersten Schaltung (nicht gezeigt) verbunden. Diese erste Schaltung erlaubt das Überprüfen der Position des Fingers, wenn dieser die Sensoroberfläche 35 berührt. Das Überprüfen der Fingerposition sollte
vorgenommen werden, bevor der Fingerabdruck
mittels der druckempfindlichen erhabenen Segmente 22 der Kontakteinrichtung 3 untersucht
wird. Falls der Finger nicht korrekt in der Mitte der
Abtastoberfläche positioniert ist, stellt die erste
Schaltung diesen Fehler fest und aktiviert eine
Alarmeinrichtung, beispielsweise eine optische
oder akustische Einrichtung, wie beispielsweise
eine Lampe bzw. einen Lautsprecher. Der Alarm
kann die Korrektur der Fingerposition veranlassen.

Ein einfaches Ausführungsbeispiel für die erste
Schaltung oder den Positionsindikator (nicht gezeigt) könnte einen Schalter und eine optische Signaleinrichtung (beispielsweise eine kleine Lampe oder eine lichtemittierende Diode LED) umfassen, die jedem Paar von Infrarot-(IR)-Sendern 31
und Infrarot-(IR)-Empfängern 32 zugeordnet
wäre. Die optische Signaleinrichtung kann auf
dem Rahmen nahe dem Infrarot-(IR)-Sender/In-
frarot-(IR)-Empfänger-Paar 31, 32 angeordnet
sein, dem sie zugeordnet ist. Fall der Finger nicht
nahe einem speziellen Infrarot-(IR)-Sender/In-
frarot-(IR)-Empfänger-Paar 31, 32 positioniert
wird, wird der Schalter wegen des Fehlens eines
genügend starken Signals nicht durch den In-
frarot-(IR)-Empfänger 32 betätigt. Deshalb wird
die zugeordnete optische Signaleinrichtung nicht
aus einer geeigneten Quelle mit Energie versorgt.
Eine nicht mit Energie versorgte Signaleinrichtung
zeigt an, dass die Position des Fingers in bezug auf
die spezielle Signaleinrichtung nicht korrekt ist
und dass eine Korrektur erforderlich ist. Falls jedoch der Finger korrekt positioniert wurde, stellt
der Infrarot-(IR)-Empfänger 32 genügend Leistung zur Verfügung, um den Schalter zu betätigen, welcher daraufhin die Signaleinrichtung veranlasst, ein optisches Signal, beispielsweise ein
Flackersignal, abzugeben. Dieses Signal zeigt an,
dass die Fingerposition nun korrekt ist.

Die Infrarot-(IR)-Sender 31 und die Infrarot-
(IR)-Empfänger 32 können ausserdem mit einer
zweiten Schaltung (nicht gezeigt) verbunden
sein. Diese zweite Schaltung ist eine Schutzschaltung, die eine Entscheidung darüber zulässt, ob ein
menschlicher Finger oder ein künstlicher Gegenstand die Sensoroberfläche berührt. Aus dem
Lichtsignal, das durch den Infrarot-(IR)-Empfän-
ger 32 aufgenommen wird, wird die Pulsfrequenz
der Person, die den Sensor berührt, bestimmt. Lediglich in einem Fall, in dem die zweite Schaltung
herausgefunden hat, dass eine Pulsfrequenz vorherrscht und dass demzufolge ein lebender Finger
den Fingerabdrucksensor berührt, wird das Fingerabdruck-Auswertungsverfahren einwandfrei
in Gang gesetzt.

Die zeite Schaltung kann ein Tiefpassfilter enthalten, das Gleichspannung und hohe Frequenzen
sperrt. Sobald das Filter erkennt, dass eine niedrige
Frequenz, die in dem Frequenzband des menschlichen Pulsschlages enthalten ist, vorliegt, steuert
das Ausgangssignal des Filters eine Torschaltung
auf, die in der Folge den Identifikationsvorgang in
Gang setzt.

In Fig. 12 ist ein weiteres Ausführungsbeispiel
für den Fingerabdrucksensor gezeigt. Während die
Sensoroberfläche 35 des Sensors gemäss Fig. 11
eben ist, ist die Sensoroberfläche 35 des Sensors
gemäss Fig. 12 gewölbt. Das Profil der Sensoroberfläche 35 ist an die Geometrie des Fingers angepasst. Auch in diesem Ausführungsbeispiel sind
auf dem Rahmen des Fingerabdrucksensors verschiedene Infrarot-(IR)-Sender 31 und Infrarot-
(IR)-Empfänger 32 angeordnet.

**Patentansprüche**

1. Fingerabdrucksensor zum Umsetzen der Fingerabdruckinformation eines zu untersuchenden
Fingers in ein elektrisches Ausgangssignal, dadurch gekennzeichnet, dass eine Kontakteinrichtung (3) vorgesehen ist, die aus einem piezoelektrischen Material besteht und Kontaktflächen zum Ausüben eines Kontaktdruckes auf sie mittels des Fingers (2) hat, wobei eine Änderung der Dichte von elektrischen Ladungen auf den Kontaktflächen entsprechend dem Fingerabdruckmuster des Fingers (2) stattfindet, und dass eine elektrische Ladungsmesseinrichtung (4) vorgesehen ist, die elektrische Ausgangssignale in Übereinstimmung mit der Verteilung der Ladungen auf zumindest einer der Kontaktflächen abgibt.

2. Fingerabdrucksensor nach Anspruch 1, dadurch gekennzeichnet, dass die Kontakteinrichtung (3) ein piezoelektrisches Polymer enthält.

3. Fingerabdrucksensor nach Anspruch 2, dadurch gekennzeichnet, dass das piezoelektrische Polymer ein Polyvinylidenfluorid ist.

4. Fingerabdrucksensor nach Anspruch 2, dadurch gekennzeichnet, dass die Kontakteinrichtung (3) eine Schicht eines piezoelektrischen Polymers darstellt, die eine erste und eine zweite Oberfläche aufweist, und dass die erste Oberfläche die Kontaktfläche zum Ausüben eines Kontaktdruckes auf sie enthält und die zweite Oberfläche elektrische Mittel zum Bestimmen der Verteilung von elektrischen Ladungen.

5. Fingerabdrucksensor nach Anspruch 2, dadurch gekennzeichnet, dass eine Belegung aus einem piezoelektrischen Polymer vorgesehen ist, das elektrische Dipole aufweist, deren Achsen senkrecht zu der Ebene der Belegung ausgerichtet sind, dass eine Anordnung von elektrischen Sensorelementen (5) an der Belegung angebracht ist und dass Mittel zum Bestimmen der Ladung auf den Sensorelementen (5) vorgesehen sind, um das elektrische Ausgangssignal zu bilden.

6. Fingerabdrucksensor nach Anspruch 5, dadurch gekennzeichnet, dass die Sensorelemente (5) in Form einer Sensormatrix angeordnet sind.

7. Fingerabdrucksensor nach Anspruch 5, dadurch gekennzeichnet, dass die elektrischen Sensorelemente in einer Anordnung ähnlich der Struktur eines Fingerabdrucks angeordnet sind.

8. Fingerabdrucksensor nach Anspruch 1, dadurch gekennzeichnet, dass die Kontakteinrichtung (3) eine Platte aus einem piezoelektrischen Material umfasst, deren Kontaktfläche strukturiert ist und eine Vielzahl von erhabenen Segmenten (22) aufweist.

9. Fingerabdrucksensor nach Anspruch 8, dadurch gekennzeichnet, dass die erhabenen Segmente (22) mit Elektroden (11, 12) versehen sind.

10. Fingerabdrucksensor nach Anspruch 8, dadurch gekennzeichnet, dass ein Isoliermaterial zwischen jeweils benachbarten erhabenen Segmenten (22) vorgesehen ist.

11. Fingerabdrucksensor nach Anspruch 1, dadurch gekennzeichnet, dass auf der Kontaktfläche eine dünne Schutzbeschichtung vorgesehen ist.

12. Fingerabdrucksensor nach Anspruch 1, dadurch gekennzeichnet, dass eine Vielzahl von metallischen Elektroden (11, 12) auf der Kontaktfläche vorgesehen ist, um die elektrischen Ladungen von der Oberfläche abgreifen zu können.

13. Fingerabdrucksensor nach Anspruch 1, dadurch gekennzeichnet, dass die Kontakteinrichtung (3) aus einem piezoelektrischen Keramikwerkstoff besteht.

14. Fingerabdrucksensor nach Anspruch 13, dadurch gekennzeichnet, dass der piezoelektrische Keramikwerkstoff ein Bleizirkonattitanatkeramikwerkstoff ist.

15. Fingerabdrucksensor nach Anspruch 14, dadurch gekennzeichnet, dass der Keramikwerkstoff Bariumtitanat enthält.

16. Fingerabdrucksensor nach Anspruch 14, dadurch gekennzeichnet, dass der Keramikwerkstoff Triglyzinsulfat enthält.

17. Fingerabdrucksensor nach Anspruch 12, dadurch gekennzeichnet, dass die Elektroden (11, 12) voneinander durch ein Isoliermaterial getrennt sind.

18. Fingerabdrucksensor nach Anspruch 1, dadurch gekennzeichnet, dass elektrische Mittel vorgesehen sind, die einen CCD-Scanner zum Bestimmen der Verteilung der elektrischen Ladungen enthalten.

19. Fingerabdrucksensor nach Anspruch 1, dadurch gekennzeichnet, dass auf einem Rahmen der Kontakteinrichtung (3) Lichtemitter und Lichtempfänger verteilt angeordnet sind und dass diese Elemente ein Ausgangssignal liefern, wenn der Finger (2), der untersucht werden soll, sich der Kontaktoberfläche nähert.

## Claims

1. A fingerprint sensor for converting the fingerprint information of a finger to be examined into an electrical output signal, characterized in that a contact device (3) is provided which consists of a piezo-electric material and has contact surfaces for the exertion of a contact pressure thereon by means of a finger (2), whereby a change of the density of electric charges on the contact surfaces in accordance with the fingerprint pattern of the finger (2) is effected, and that an electric charge measuring device (4) is provided which emits electrical output signals in conformity with the distribution of the charges on at least one of the contact surfaces.

2. A fingerprint sensor as claimed in claim 1, characterized in that the contact device (3) contains a piezo-electric polymer.

3. A fingerprint sensor as claimed in claim 2, characterized in that the piezo-electric polymer is a polyvinylidene fluoride.

4. A fingerprint sensor as claimed in claim 2, characterized in that the contact device (3) exhibits a layer of a piezo-electric polymer which has a first and a second surface, and that the first surface includes the contact surface for the exertion of a contact pressure thereon, and the second surface includes electrical means for determining the distribution of electric charges.

5. A fingerprint sensor as claimed in claim 2, characterized in that a coating made of a piezo-electric polymer is provided, which has electric dipoles, the axes of which are aligned at right angles to the plane of the coating, that an arrangement of electrical sensor elements (5) is applied to the coating, and that means are provided for determining the charge on the sensor elements (5) in order to form the electric output signal.

6. A fingerprint sensor as claimed in claim 5, characterized in that the sensor elements (5) are arranged in the form of a sensor matrix.

7. A fingerprint sensor as claimed in claim 5, characterized in that the electrical sensor elements are arranged in an arrangement similar to the structure of a fingerprint.

8. A fingerprint sensor as claimed in claim 1, characterized in that the contact device (3) comprises a plate made of a piezo-electric material, the contact surface of which is structured and exhibits a plurality of raised segments (22).

9. A fingerprint sensor as claimed in claim 8, characterized in that the raised segments (22) are provided with electrodes (11, 12).

10. A fingerprint sensor as claimed in claim 8, characterized in that an insulating material is arranged between adjacent raised segments (22).

11. A fingerprint sensor as claimed in claim 1, characterized in that a thin protective coating is arranged on the contact surface.

12. A fingerprint sensor as claimed in claim 1, characterized in that a plurality of metal electrodes (11, 12) are arranged on the contact surface so as to be able to pick up the electric charges from the surface.

13. A fingerprint sensor as claimed in claim 1, characterized in that the contact device (3) consists of a piezo-electric ceramic material.

14. A fingerprint sensor as claimed in claim 13, characterized in that the piezo-electric ceramic material is a lead zirconate and titanate ceramic material.

15. A fingerprint sensor as claimed in claim 14, characterized in that the ceramic material contains barium titanate.

16. A fingerprint sensor as claimed in claim 14, characterized in that the ceramic material contains triglycine sulphate.

17. A fingerprint sensor as claimed in claim 12, characterized in that the electrodes (11, 12) are separated from one another by an insulating material.

18. A fingerprint sensor as claimed in claim 1, characterized in that electrical means are provided which include a CCD scanner for determining the distribution of the electric charges.

19. A fingerprint sensor as claimed in claim 1, characterized in that light emitters and light receivers are distributed on a frame portion of the contact device (3), and that these elements supply an output signal when the finger (2) to be examined approaches the contact surface.

## Revendications

1. Capteur d'empreintes digitales servant à convertir l'information de l'empreinte digitale d'un doigt devant être examiné en un signal électrique de sortie, caractérisé par le fait qu'il est prévu un dispositif de contact (3) qui est constitué en un matériau piézo-électrique et possède des surfaces de contact en vue de permettre l'application d'une pression de contact sur celles-ci au moyen du doigt (2), une modification de la densité des charges électriques sur les surfaces de contact intervenant en fonction du modèle de l'empreinte digitale du doigt (2), et qu'il est prévu un dispositif électrique (4) de mesure des charges, qui délivre des signaux électriques de sortie en conformité avec la distribution des charges sur au moins une des surfaces de contact.

2. Capteur d'empreintes digitales selon la revendication 1, caractérisé par le fait que le dispositif de contact (3) contient un polymère piézo-électrique.

3. Capteur d'empreintes digitales selon la revendication 2, caractérisé par le fait que le polymère piézo-électrique est un fluorure de polyvinylidène.

4. Capteur d'empreintes digitales selon la revendication 2, caractérisé par le fait que le dispositif de contact (3) est constitué par une couche d'un polymère piézo-électrique qui possède une première et une seconde surface, que la première surface contient la surface de contact permettant d'exercer une pression de contact, et que la seconde surface contient des moyens électriques pour déterminer la distribution des charges électriques.

5. Capteur d'empreintes digitales selon la revendication 2, caractérisé par le fait qu'il est prévu un revêtement formé d'un polymère piézo-électrique qui possède des dipôles électriques dont les axes sont orientés perpendiculairement au plan de la couche, qu'un agencement d'éléments électriques détecteurs (5) est disposé sur le revêtement et qu'il est prévu des moyens pour déterminer la charge sur les éléments détecteurs (5) afin de former le signal électrique de sortie.

6. Capteur d'empreintes digitales selon la revendication 5, caractérisé par le fait que les éléments détecteurs (5) sont disposés sous la forme d'une matrice de détecteurs.

7. Capteur d'empreintes digitales selon la revendication 5, caractérisé en ce que les éléments détecteurs électriques sont disposés selon un agencement semblable à la structure d'une empreinte digitale.

8. Capteur d'empreintes digitales selon la revendication 1, caractérisé par le fait que le dispositif de contact (3) contient une plaque constituée en un matériau piézo-électrique dont la surface de contact est structurée et qui comporte un grand nombre de segments en relief (22).

9. Capteur d'empreintes digitales suivant la revendication 8, caractérisé par le fait que les segments en relief (22) sont munis d'électrodes (11, 12).

10. Capteur d'empreintes digitales selon la revendication 8, caractérisé en ce qu'un matériau isolant est prévu entre deux segments en relief (22) respectivement voisins.

11. Capteur d'empreintes digitales selon la revendication 1, caractérisé par le fait qu'un revêtement protecteur mince est prévu sur la surface de contact.

12. Capteur d'empreintes digitales selon la revendication 1, caractérisé par le fait qu'il est prévu un grand nombre d'électrodes métalliques (11, 12) sur la surface de contact afin de pouvoir prélever les charges électriques de la surface supérieure.

13. Capteur d'empreintes digitales selon la revendication 1, caractérisé en ce que le dispositif de contact (3) est constitué en un matériau céramique piézo-électrique.

14. Capteur d'empreintes digitales selon la revendication 13, caractérisé en ce que le matériau céramique piézo-électrique est un matériau céramique à base de zirconate et de titanate de plomb.

15. Capteur d'empreintes digitales selon la revendication 14, caractérisé en ce que le matériau céramique contient du titanate de baryum.

16. Capteur d'empreintes digitales selon la revendication 14, caractérisé par le fait que le matériau céramique contient du sulfate de triglycine.

17. Capteur d'empreintes digitales selon la revendication 12, caractérisé par le fait que les électrodes (11, 12) sont séparées les unes des autres par un matériau isolant.

18. Capteur d'empreintes digitales selon la revendication 1, caractérisé par le fait qu'il est prévu des moyens électriques qui contiennent un dispositif d'exploration CCD pour la détermination de la distribution des charges électriques.

19. Capteur d'empreintes digitales selon la revendication 1, caractérisé en ce que des émetteurs de lumière et des récepteurs de lumière sont répartis sur un cadre du dispositif de contact (3) et que ces éléments délivrent un signal de sortie lorsque le doigt (2) à examiner se rapproche de la surface de contact.

0 044 489

FIG. 1

FIG. 2

FIG. 3

11

FIG. 4

FIG. 5

FIG. 6

FIG. 7

FIG. 8

FIG. 9

FIG. 10

FIG. 11

FIG. 12